# EUROPEAN PATENT APPLICATION

(11) **EP 4 574 152 A1**
(43) Date of publication of application: **25.06.2025**
(21) Application number: 23220110.3
(22) Date of filing: 22.12.2023
(51) Int. Cl.: A61K 31/501, A61P 25/02, A61P 9/00, A61P 25/04, A61P 25/06, A61P 25/08, A61P 25/14, A61P 25/18, A61P 25/22, A61P 25/30, A61P 27/16

(54) **POTASSIUM CHANNEL ACTIVATORS FOR USE IN THE TREATMENT OF EPILEPTIC DISORDERS**

(71) Applicant: Fraunhofer-Gesellschaft zur Förderung der angewandten Forschung e.V., 80686 München (DE)
(72) Inventor: Gribbon, Phil, 80686 Munich (DE); Taglialatela, Maurizio, 80138 Naples (IT); Miceli, Francesco, 80138 Naples (IT); Carotenuto, Lidia, 80138 Naples (IT); Weckhuysen, Sarah, 2610 Antwerp (BE); Leo, Antonio, 88100 Catanzaro (IT); Citraro, Rita, 88100 Catanzaro (IT); De Sarro, Giovanbattista, 88100 Catanzaro (IT); KEMINER, Oliver, 80696 Munich (DE)
(74) Representative: Kuttenkeuler, David

(57) **Abstract**

The invention is based on the repurposing of a clinically safe compound as a potassium channel activator for treating hyperexcitability related disorders. The invention also relates to a pharmaceutical composition comprising a compound of the present invention, as well as a method of treating hyperexcitability related disorders using the disclosed compounds or a pharmaceutical composition of the invention.

## Description

### FIELD OF THE INVENTION

The invention is based on the repurposing of a clinically safe compound as a potassium channel activator for treating hyperexcitability related disorders. The invention also relates to a pharmaceutical composition comprising a compound of the present invention, as well as a method of treating hyperexcitability related disorders using the disclosed compounds or a pharmaceutical composition of the invention.

### DESCRIPTION

Epileptic disorders, characterized by enduring predisposition to generate seizures, are among the most frequent neurological diseases, with a lifetime prevalence estimated between 1 and 2%. Treatment of epileptic disorders often relies on the use of antiseizures medications (ASMs), administered chronically with the intent of preventing the occurrence of seizures. However, seizures in more than 30% of the patients are not adequately controlled with currently-available ASMs, resulting in the development of pharmacoresistant epilepsy (PRE). In particular, a patient is deemed to suffer from PRE when seizures are inadequately controlled despite treatment with ≥2 ASMs properly selected based on the patient's history and used at adequate doses.

Long-term outcome studies suggest that the chance of success in controlling seizures in PRE patients upon introduction of additional drugs in the therapeutic schemes becomes progressively less likely. Lack of seizure control is associated with significant comorbidities, including a higher risk of "sudden unexpected death in epilepsy" (SUDEP), and, in children, with failure to achieve neurodevelopmental milestones, thus significant intellectual disabilities may occur. Although treatment approaches different from ASMs, such as resective surgery and/or ketogenic diet may help to control seizures in some patients with PRE, not all patients qualify for surgery and long-term exposure to ketogenic diet is associated with severe side effects.

Currently, available ASMs can be classified on the basis of their mechanism of action, the most exploited being: 1. voltage-gated sodium channel blockade, 2. increased GABAergic neurotransmission and 3. voltage-gated calcium channels blockade. It is commonly believed that treatment with ASMs endowed with different, newer, and alternative mechanisms of action may reduce PRE, thus the urgent need to design innovative pharmacological strategies to combat the hyperexcitability process underlying seizure disorders.

Activation of potassium channels has recently emerged as one of the most promising mechanisms for the anticonvulsive effects of novel ASMs. Among voltage-gated (Kv) potassium channel, those encoded by the KCNQ subfamily have received the widest attention in the past decades. KCNQ genes (KCNQ1-5) encode for five members (Kv7.1-Kv7.5) of Kv7 subunits, each characterized by distinct tissue distribution and pathophysiological role. Kv7.1 subunits are expressed in the heart, where they represent the main molecular component of the ventricular repolarization current known as I_{Ks}; instead, Kv7.2 and Kv7.3 subunits are mainly expressed in the nervous system (CNS) where they underly the M-current, a repolarizing current active at resting membrane potential and limiting the neuronal spike discharge rate; Kv7.4 is mainly expressed in the inner ear and in the visceral, vascular and pulmonary smooth muscles, being involved in the regulation of smooth muscle tone; finally, Kv7.5 is mainly expressed in the CNS, vascular smooth and skeletal muscles.¹¹

The pivotal role of neuronal Kv7 channels (mainly Kv7.2 and Kv7.3, but with a contribution also from Kv7.5) in neuronal excitability is evidenced by the occurrence of convulsive disorders with high phenotypic variability in humans, caused by specific mutations of their corresponding genes.

Given the prominent role played by Kv7 channels in controlling neuronal excitability and considering that neuronal hyperexcitability is a common feature of neuropsychiatric disorders such as epilepsy, neuropathic pain, ischemic stroke, amyotrophic lateral sclerosis, and several other neurodegenerative diseases, pharmacological modulation of Kv7 channels appears as a rational approach to treat these conditions.

As a matter of fact, two neuronal Kv7 activators, flupirtine and retigabine, have already been approved and clinically used. Flupirtine was the first compound identified as a Kv7 activator, even though its development preceded the discovery of these channels. Flupirtine is a non-opioid analgesic, approved in 1984 in Europe, but whose marketing authorization was withdrawn by the EMA in 2018 because of its severe liver toxicity.

Limited preclinical and clinical studies performed in the '80s showed that flupirtine was also provided with anticonvulsant effects in animal models and in patients with PRE; thus, optimization of the flupirtine molecule to enhance its antiepileptic activity led to synthesis and preclinical characterization of retigabine, (ezogabine in the US), which is now considered the prototype Kv7 opener.

The anticonvulsant efficacy of retigabine has been demonstrated in numerous animal models of epilepsy, and is due to the suppression of neuronal hyperexcitability consequent to a unique mode of activation of Kv7.2/7.3 channels, consisting in a shift in current activation threshold toward more hyperpolarized potentials, and an enhancement of the Kv7.2/7.3 maximal current.¹⁶ Its mechanism of action, different from classic ASMs, makes retigabine particularly effective for treatment of patients suffering from PRE. In 2011 retigabine was approved as adjunctive treatment in adults with partial-onset seizures. However, long-term use of retigabine causes blue discoloration of muco-cutaneous tissues and of the retina, due to the photo-induced formation and accumulation of retigabine dimers, especially in melanin-rich tissues. Mainly because of these adverse events, clinical use of retigabine progressively declined, leading to its withdrawal from the market in 2017.

Therefore, despite several studies validating the pharmacological activation of neuronal Kv7 channels as an effective mechanism for the treatment of epileptic disorders and of other conditions characterized by neuronal hyperexcitability, no Kv7 activator is currently available for clinical use.

Several industrial and academic groups are currently developing new Kv7 activators. A number of analogues of retigabine/flupirtine with improved physico-chemical, pharmacokinetic, or pharmacodynamic properties, as well as new Kv7 openers originating from pharmacophoric structures distinct from the two parental molecules have been investigated over the past two decades.

Among newly synthesized retigabine analogues, several are more potent than the parental molecule in activating Kv7.2 and Kv7.3 channels *in vitro,* some also showing higher efficacy and potency as anticonvulsants in animal models of epilepsy. In addition, to overcome the tissue discoloration observed upon long-term use of retigabine, different synthetic strategies have been successfully applied to develop retigabine analogues unable to generate the molecular byproducts responsible for this adverse event, in some cases also obtaining potent anticonvulsants in animal models of seizures.

In addition to retigabine analogues, several Kv7 activators with a chemical structure different from retigabine have also been described; these belong to the chemical classes of acrylamides, benzamides, fenamates, or present unique structures not included in any of the mentioned classes. Acrylamides bind Kv7 channels in the same region in the channel pore occupied by retigabine, and, once they were demonstrated effective in reducing neuronal excitability *in vitro,* they were investigated for possible efficacy in migraine and neuropathic pain. Fenamates, such as the nonsteroidal anti-inflammatory drug diclofenac, activate Kv7.2 and Kv7.3 channels and exhibit anticonvulsant activity in animal models of seizures. A few diclofenac derivatives, designed to abolish COX inhibitory activity while retaining the Kv7-opening property, were effective in reducing neuronal activity *in vitro* but none of these molecules has been tested *in vivo.*

Benzamides show a binding site and a mechanism of action distinct from retigabine. Various benzamides were found to be more potent than retigabine *in vitro* and exerted antiseizures effect in several animal model of epilepsy.

Several other chemotypes have been investigated *in vitro* as Kv7 activators, both synthesized or natural compounds. Among these, some molecules also showed anticonvulsant activity in animal models, one (KB 3061 or BHV-7000) was able to revert *in vitro* the effect of pathogenic KCNQ mutations.

Despite the promising results obtained in preclinical models, few Kv7 activators have reached the clinical phase of investigation. Among these:
- the retigabine analogue HN37 progressed to clinical trials in China for epilepsy treatment;

The compound XEN1101 is currently being studied in three different phase II/III clinical trials: 1. for the treatment of focal-onset seizures (ClinicalTrials.gov Identifier: NCT05614063); 2. as adjunctive therapy in primary generalized tonic-clonic seizures (ClinicalTrials.gov Identifier: NCT05667142); 3. for the treatment of major depressive disorder (ClinicalTrials.gov Identifier: NCT04827901).

The compound BHV-7000 passed the phase I safety trials and a phase II/III trial for focal epilepsy and bipolar disorder was announced for 2023.

Moreover, a new paediatric formulation of retigabine from Xenon Pharmaceuticals, XEN496, was tested in subjects with KCNQ-related disorders, in a clinical trial terminated in 2023 whose results have not yet been released (ClinicalTrials.gov Identifier: NCT04639310).

The gap between the number of new Kv7 openers investigated in preclinical studies and those undergoing clinical investigation is consistent with the high risk of failure of developing a drug from a new chemical entity.

Ideally, if an alternative drug could be identified, which in more advanced stages of development, often clinically-investigated or approved, for similar, if not superior effect, the risk of failure, the economic investment, and the timeframe to start treating patients would be reduced.

Thus, it is an object of the invention to repurpose a previously known antipsychotic drug as a potassium channel activator for treating Kv7-associated disorders.

### BRIEF DESCRIPTION OF THE INVENTION

Generally, and by way of brief description, the main aspects of the present invention can be described as follows:
In **a first aspect,** the invention pertains to a compound in the treatment of a Kv7-associated disorder in a subject, wherein the compound is selected from a compound of formula (I) as defined herein below, or a pharmaceutically acceptable salt, hydrate or solvate thereof, or a stereoisomeric form thereof.
In **a second aspect,** the invention pertains a method of treatment of a neurological disease in a subject, the method comprising the steps of administering to the subject a therapeutically effective amount of a compound according to the present invention, wherein the subject suffers from a neurological disorder that is not a psychotic disorder, but wherein subject experienced or is experiencing convulsive seizures.
In **a third aspect,** the invention pertains to a pharmaceutical composition, comprising a compound of the present invention, together with a pharmaceutically acceptable carrier and/or excipient.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the object is solved by providing a compound for use in the treatment of a Kv7 associated disorder in a subject, wherein the compound is selected from a compound of formula (I), or a pharmaceutically acceptable salt, hydrate or solvate thereof, or a stereoisomeric form thereof, 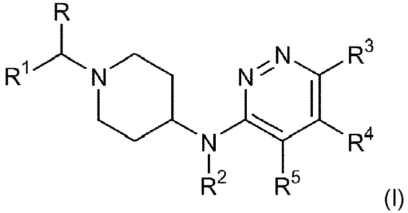 wherein
R is H or C₁₋₆alkyl;
R¹ is phenyl; phenyl substituted with 1, 2 or 3 substituents each independently selected from the group consisting of H, halogen, cyano, C₁₋₄-alkyl, C₁₋₄-alkyloxy, perfluoro-C₁₋₄-alkyl, diC₁₋₄-alkylamino; thienyl; thienyl substituted with 1 or 2 substituents selected from the group consisting of halo and C₁₋₄-alkyl; C₃₋₈-cycloalkyl; or C₅₋₇-cycloalkenyl;
R² is H or C₁₋₆-alkyl;
R³ is halogen, C₁₋₄-alkyl or perfluoro-C₁₋₄-alkyl; and
R⁴ and R⁵ are each independently selected from H or Halogen.

In one preferred embodiment of the compound for use according to the present invention, said R³ is trifluoromethyl; and R, R⁴ and R⁵ are H.

In another preferred embodiment of the compound for use according to the present invention, said R is H or methyl.

In another preferred embodiment of the compound for use according to the present invention, said R1 is 4-fluorophenyl or 3 ,4-difiuorophenyl.

In another preferred embodiment, the compound for use according to the present invention is *N*-[1-[(3,4-difluorophenyl)methyl]-4-piperidinyl]-6-(trifluoromethyl)-3-pyridazinamine, or a compound having the following formula (II)

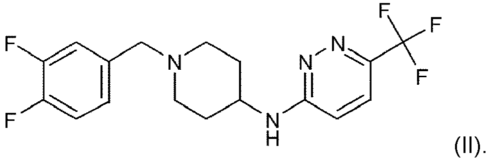

In another preferred embodiment, the compound for use according to the present invention is compound is N-[I-(4-fluorobenzyl)piperidin-4-yl]-6-(trifluoromethyl)pyridazin-3-amine, or is a compound according to formula (III)

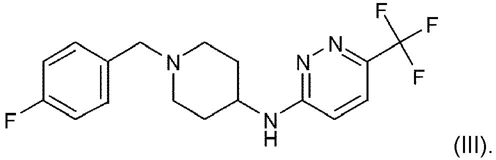

In another preferred embodiment, the compound for use according to the present invention is compound is N-[I-(4-fluorobenzyl)piperidin-4-yl]-6-(trifluoromethyl)pyridazin-3-amine, or is a compound according to formula (IV)

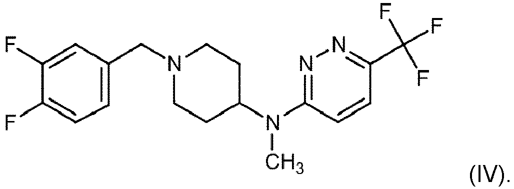

In another preferred embodiment, the compound for use according to the present invention is compound is N-[I-(4-fluorobenzyl)piperidin-4-yl]-6-(trifluoromethyl)pyridazin-3-amine, or is a compound according to formula (V)

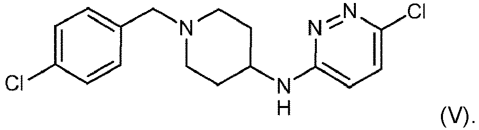

In another preferred embodiment, the compound for use according to the present invention is compound is N-[I-(4-fluorobenzyl)piperidin-4-yl]-6-(trifluoromethyl)pyridazin-3-amine, or is a compound according to formula (VI)

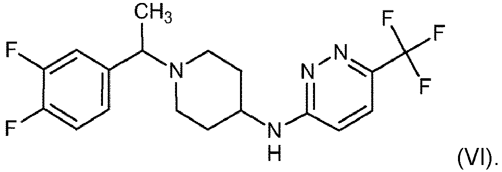

The term "alkyl" refers to a monoradical of a saturated straight or branched hydrocarbon. Preferably, the alkyl group comprises from 1 to 12 (such as 1 to 10) carbon atoms, i.e., 1, 2, 3, 4, 5, 6, 7, 8, 9, 10, 11, or 12 carbon atoms (such as 1, 2, 3, 4, 5, 6, 7, 8, 9, or 10 carbon atoms), more preferably 1 to 8 carbon atoms, such as 1 to 6 or 1 to 4 carbon atoms. Exemplary alkyl groups include methyl, ethyl, propyl, iso-propyl (also called 2-propyl or 1-methylethyl), butyl, isobutyl, tert-butyl, n-pentyl, iso-pentyl, sec-pentyl, neo-pentyl, 1,2-dimethyl-propyl, iso-amyl, n-hexyl, iso-hexyl, sec-hexyl, n-heptyl, iso-heptyl, n-octyl, 2-ethyl-hexyl, n-nonyl, n-decyl, n-undecyl, n-dodecyl, and the like. A "substituted alkyl" means that one or more (such as 1 to the maximum number of hydrogen atoms bound to an alkyl group, e.g., 1, 2, 3, 4, 5, 6, 7, 8, 9, or up to 10, such as between 1 to 5, 1 to 4, or 1 to 3, or 1 or 2) hydrogen atoms of the alkyl group are replaced with a substituent other than hydrogen (when more than one hydrogen atom is replaced the substituents may be the same or different). Preferably, the substituent other than hydrogen is a 1st level substituent, a 2nd level substituent, or a 3rd level substituent as specified herein, such as halogen, -OH, -NH2, -NHCH3, -N(CH3)2, -CN, -OCH3, -OCF3, or optionally substituted aryl. Examples of a substituted alkyl include trifluoromethyl, 2,2,2-trichloroethyl, 2-hydroxyethyl, 2-aminoethyl, 2-(dimethylamino)ethyl, arylalkyl (also called "aralkyl", e.g., benzyl, chloro(phenyl)methyl, 4-methylphenylmethyl, (2,4-dimethylphenyl)methyl, o-fluorophenylmethyl, 2-phenylpropyl, 2-, 3-, or 4-carboxyphenylalkyl), or heteroarylalkyl (also called "heteroaralkyl").

Cₓ₁₋ₓ₂-alkyl shall mean an alkyl group as described above with a chain length or in other words, with the number of carbon atom between the number X1 and X2 as denoted in the subscript. For example, the term "C₁₋₃alkyl" refers to an alkyl group with a chain length of between 1 to 3 carbons.

The term "halogen" or "halo" means fluoro, chloro, bromo, or iodo.

The term cyano shall mean a carbon atom with three single bonds to a nitrogen atom and as a nitrogen atom bonded to three carbon atoms.

The term alkyloxy shall refer to an alkyl group as described above which is singularly bonded to oxygen.

The term perfluoro shall refer to a chemical group, in which all C-H bonds have been replaced by C-F bonds.

The term alkylamino shall mean alkyl group as described above attached to the remainder of a molecule via nitrogen.

The term The term "cycloalkyl" represents cyclic non-aromatic versions of "alkyl" with preferably 3 to 14 carbon atoms, such as 3 to 12 or 3 to 10 carbon atoms, i.e., 3, 4, 5, 6, 7, 8, 9, 10, 11, 12, 13, or 14 carbon atoms (such as 3, 4, 5, 6, 7, 8, 9, or 10 carbon atoms), more preferably 3 to 7 carbon atoms. Exemplary cycloalkyl groups include cyclopropyl, cyclopropenyl, cyclobutyl, cyclobutenyl, cyclopentyl, cyclopentenyl, cyclohexyl, cyclohexenyl, cycloheptyl, cycloheptenyl, cyclooctyl, cyclooctenyl, cyclononyl, cyclononenyl, cylcodecyl, cylcodecenyl, and adamantyl. The term "cycloalkyl" is also meant to include bicyclic and tricyclic versions thereof. If bicyclic rings are formed it is preferred that the respective rings are connected to each other at two adjacent carbon atoms, however, alternatively the two rings are connected via the same carbon atom, i.e., they form a spiro ring system or they form "bridged" ring systems. Preferred examples of cycloalkyl include C3-8-cycloalkyl, in particular cyclopropyl, cyclobutyl, cyclopentyl, cyclohexyl, cycloheptyl, cyclooctyl, spiro[3,3]heptyl, spiro[3,4]octyl, spiro[4,3]octyl, bicyclo[4.1.0]heptyl, bicyclo[3.2.0]heptyl, bicyclo[2.2.1] heptyl, bicyclo[2.2.2]octyl, bicyclo[5.1.0]octyl, and bicyclo[4.2.0]octyl. Cycloalkyl does not encompass fullerenes. A "substituted cycloalkyl" means that one or more (such as 1 to the maximum number of hydrogen atoms bound to a cycloalkyl group, e.g., 1, 2, 3, 4, 5, 6, 7, 8, 9, or up to 10, such as between 1 to 5, 1 to 4, or 1 to 3, or 1 or 2) hydrogen atoms of the cycloalkyl group are replaced with a substituent other than hydrogen (when more than one hydrogen atom is replaced the substituents may be the same or different). Preferably, the substituent other than hydrogen is a 1st level substituent, a 2nd level substituent, or a 3rd level substituent as specified herein, such as halogen, -CN, nitro, -OR11 (e.g., -OH), -SR11 (e.g., -SH), -N(R12)(R13) (e.g., -NH ), =X (e.g., =O, =S, or =NH), alkyl (e.g., C₁₋₆alkyl). Examples of a substituted cycloalkyl include oxocyclohexyl, oxocyclopentyl, fluorocyclohexyl, and oxocyclohexenyl.

The compound according to the present invention was originally developed as a highly selective dopamine D2 receptor antagonist with a rapid dissociation rate (in WO2007048779), exerting an antipsychotic effect without motor side effects.

The inventor screened extensively a compound library comprising a large number of compounds that have already been proven clinically safe and made the surprising invention that there is indeed specific Kv7 activator (the compound of the present invention) that is already safe for clinical use (example 1).

Additionally, the compound of the present invention can be proven to be an activator of neuronal Kv7 channels, particularly Kv7.2/7.3 channels and, by this mechanism of action, effectively controls seizures in two different animal models of epilepsy (example 2 and 4). A Kv7 channel preferred in accordance with the invention is a protein having an amino acid sequence set forth in SEQ ID NO: 1.

An advantageous embodiment lies in the fact that the compound according to the present invention has already been confirmed as safe for human use, did not cause any serious side effects when tested in a 12-week clinical trial and showed favorable pharmacodynamic features such as good brain penetration.

Additionally, the compound for use according to the current invention is found to interact with W234 of Kv7.2 with its piperazine ring through docking simulation data. This piperazine ring is found to form an H-bond with the indole nitrogen atom of W236 of Kv7.2 (SEQ ID NO 1). This mimics the interaction between the carbamate group of retigabine and the same W236 of Kv7.2. Of note, this W235 binding residue is located at the pore domain of Kv7.2, rationalizing it as a binding target for modulating Kv7.2 activity. In addition, two phenylalanine residues (F240 and F305) in Kv7.2, which are known to interact with the parafluoro phenyl moiety of retigabine, also interacted with the difluorophenyl moiety in the compound according to the present invention. References to amino acid positions are with regard to the amino acid sequence set forth in SEQ ID NO: 1.

Therefore, another preferred embodiment of the compound according to the present invention is a compound comprising a piperazine, or a similar moiety that may act as an acceptor of an H-bond donated by W236. References to amino acid positions are with regard to the amino acid sequence set forth in SEQ ID NO: 1.

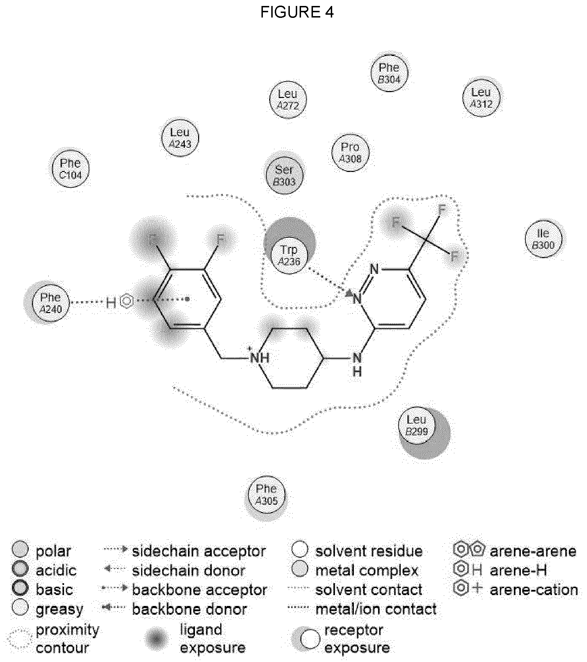

Through the docking simulation, interaction site can be modeled as is shown in figure 4. It is another preferred embodiment that a compound of the invention retains a capability to interact or at stay in contact with at least one of (preferably all of) residues F104, L242, F304, L312, I300, P308, S303, W236, L299, F305, F240 as a Kv7.2 binding site for the compound of the present invention when bound to Kv7.2. References to amino acid positions are with regard to the amino acid sequence set forth in SEQ ID NO: 1.

On top of the binding mechanism, the inventor has further confirmed the activation of Kv7 channel when in contact with the compound of the present invention. So, another important embodiment of the compound according to the present invention is its ability to modulate the activity of Kv7 channels, in particular Kv7.2/Kv7.3 (Example 2).

In another preferred embodiment, the compound of the present invention acts is an Kv7 agonist, preferably a Kv7.2- and Kv7.3-agonist. As used herein, an "agonist" shall refer to a compound that activates a biological target, in particular a protein receptor, to produce a biological effect.

In a related embodiment, the compound of the present invention interacts with a Kv7 potassium channel and thereby increases ion flux, in particular, potassium ion flux, across the Kv7 potassium channel. As used herein, the term "ion flux" refers to the movement of ions across, normally, across a membrane.

As a result, in another preferred embodiment of the compound according to the present invention, the administration of the compound to the subject reduces the occurrence, prevalence, frequency, duration and/or severity of seizures, wherein said subject suffers from a seizure inducing disease.

As used herein, the term "Kv7 channel" refers to one member of a group of voltage-gated potassium channels encoded by the KCNG genes. The Kv7 family is a group of tetrameric voltage-gated potassium channels consisting of five members Kv7.1, Kv7.2, Kv 7.3, Kv7.4, Kv7.5, whose subunits are encoded by the KCNG1-5 genes respectively. Another important embodiment of compound of the present invention is therefore its ability to induce the opening of Kv7.2 and/or Kv7.3 channels.

The Kv7 channels may be activated and allow the passing of potassium ions, for example across cellular membranes; this induced permeability of potassium ions is herein referred to as an "opening" or "activation" of the respective channel protein.

In another embodiment, the compound for use according to the invention is in salt format, and preferably wherein the salt is a dihydrochloride salt.

In another embodiment, the compound for use according to the invention is used as an antiseizures medication (ASM). As used herein, an antiseizures medication should be understood as an agent used in the treatment of seizures.

Since the activity of Kv7.2/7.3 has been linked to neurological disorder, in a second aspect, the invention pertains a method of treatment of a neurological disease in a subject, the method comprising the steps of administering to the subject a therapeutically effective amount of a compound according to the present invention, wherein the subject suffers from a neurological disorder that is not a psychotic disorder, but wherein the subject experienced or is experiencing convulsive seizures. It was surprisingly identified that the compounds defined herein show alleviating activities towards seizures *in vivo.*

Therefore, in a preferred embodiment of the method according to the present invention, the neurological disease is selected from Kv7-associated diseases. Particularly, it can be selected from disorders involving neuronal hyperexcitability, such as epileptic syndromes, neuropathic pain conditions, amyotrophic lateral sclerosis, ischemic stroke, neurodegenerative diseases, among others. The neurological disease may be for example, but is not limited to, epilepsy, including generalized epilepsy, focal epilepsy, combined generalized and focal epilepsy, unknown epilepsy, early myoclonic encephalopathy, Ohtahara syndrome, West syndrome, Dravet syndrome, myoclonic status in non-progressive encephalopathies, Lennox-Gastaut syndrome, Landau-Kleffner syndrome, and epilepsy with continuous spike waves during slow-wave sleep, migrating partial seizures in infancy, neuropathic pain, ischemic stroke, amyotrophic lateral sclerosis, Alzheimer's disease, Parkinson's disease.

The term "Kv7-associated disease" shall be understood in the context of the present invention as any disease or disorder in a given subject that is mainly caused by the malfunction of the voltage-gated potassium channel of the Kv7 (KCNQ) family.

However, as the object of the present invention relates to providing a novel treatment of neurological diseases associated with potassium channel, such as diseases associated with convulsive seizures, said neurological disease shall not encompass diseases associated with dopamine D2 receptor malfunction, in particular, central nervous system diseases associated with dopamine D2 receptor malfunction. In particular, said neurological disorder is not a neurological disorder unrelated to motor functions.

The term "treatment" shall refer to a partial or total inhibition and/or reduction of symptoms of a particular disease, such as a neurological disease, like epilepsy, in a subject, The term "prevention" shall refer to preventing or delaying the onset of a clinically evident disease, such as a neurological disease, like epilepsy, in a subject. In the context of the present invention, prevention and/or treatment shall include both preventive and/or actual treatment of the disease symptoms of a disease, such as a proliferative disease, like cancer, which can be alleviated and/or even completely removed using said treatment.

In **a third aspect,** the invention pertains to a pharmaceutical composition, comprising a compound of the present invention, together with a pharmaceutically acceptable carrier and/or excipient.

The compounds of this invention are customarily administered in the form of pharmaceutical compositions, which comprise at least one compound according to the invention, optionally together with an inert carrier (e.g. a pharmaceutically acceptable excipient) and, where appropriate, other compounds and/or drugs.

The skilled artisan is aware of suitable forms of pharmaceutical compositions, for example solid forms, such as powders, granules, tablets, in particular film tablets, lozenges, sachets, cachets, sugar-coated tablets, capsules, such as hard gelatin capsules and soft gelatin capsules, or suppositories, semisolid medicinal forms, such as ointments, creams, hydrogels, pastes or plasters, or liquid medicinal forms, such as solutions, emulsions, in particular oil-in-water emulsions, suspensions, for example lotions, injection preparations and infusion preparations. In addition, it is also possible to use liposomes or microspheres.

In a particularly preferred embodiment, a pharmaceutical composition comprising at least one compound according to the invention, a pharmaceutically acceptable salt, solvate or optical isomer thereof, is provided in form of a gel, an ointment, a salve, a cream, a tablet, a pill, a capsule, a troche, a dragée, a powder, an aerosol spray, a nasal spray, a suppository, and/or a solution.

When producing a pharmaceutical composition according to this invention, at least one compound of the present invention, is optionally mixed or diluted with one or more carriers and/or excipients. Carriers and/or excipients, according to this invention, can be solid, semisolid or liquid materials, which may serve as a vehicle, a carrier or a medium for the active compound.

In another preferred embodiment, a pharmaceutical composition according to the invention comprises the compound according to the invention in an amount effective to agonise one or more Kv7 potassium channels in a subject.

In another preferred embodiment, a pharmaceutical composition according to the invention comprises the compound according to the invention in an amount effective as an antiseizures medication (ASM).

In another preferred embodiment, a pharmaceutical composition according to the invention comprises the compound according to the invention present as dihydrochloride.

Further, a pharmaceutical composition according to this invention might further comprise at least one acceptable auxiliary substance, such as a wetting agent, an emulsifying agent, a suspending agent, a preservative, an antioxidant, an anti-irritant, a chelating agent, a coating agent, an emulsion stabilizer, a gel former, a resin, a solvent, a solubilizer, a neutralizing agent, a pigment, a silicone derivative, a binder, a filler, a drying agent, a thickener, a wax, a plasticizer, or the like.

The terms "of the present invention", "in accordance with the invention", "according to the invention" and the like, as used herein are intended to refer to all aspects and embodiments of the invention described and/or claimed herein.

Of note, while the terms "a compound of the present invention", "in accordance with the invention" and the like as used herein are intended to refer to a compound with all aspects and embodiments of the invention described and/or claimed herein, the term JNJ-37822681 shall either refer to a compound with all aspects and embodiments of the invention described and/or claimed herein, or a compound with all aspects and embodiments of the invention described and/or claimed in the patent application WO2007048779, depending on the context it is used. It would be clear to the person skilled in the art that the two share structural similarities, but are not entirely the same.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:
Item 1.**A compound for use in the treatment of a Kv7 associated disorder** in a subject, wherein the Kv7 associated disorder is characterized by the occurrence of seizures and/or is an epileptic disorder; and wherein the compound is selected from a compound of formula (I), or a pharmaceutically acceptable salt, hydrate or solvate thereof, or a stereoisomeric form thereof, wherein 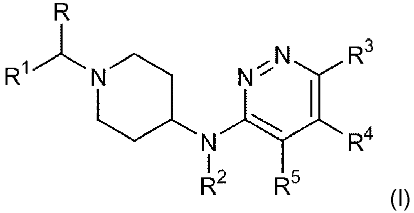
   R is H or C₁₋₆alkyl, in particular methyl;
   R¹ is phenyl; phenyl substituted with 1, 2 or 3 substituents each independently selected from the group consisting of H, halogen, cyano, C₁₋₄-alkyl, C₁₋₄-alkyloxy, perfluoro-C₁₋₄-alkyl, diC₁₋₄-alkylamino, in particular 4-fluorophenyl or 3 ,4-difiuorophenyl; thienyl; thienyl substituted with 1 or 2 substituents selected from the group consisting of halo and C₁₋₄-alkyl; C₃₋₈-cycloalkyl; or C₅₋₇-cycloal kenyl;
   R² is H or C₁₋₆-alkyl;
   R³ is halogen, C₁₋₄-alkyl or perfluoro-C₁₋₄-alkyl; and
   R⁴ and R⁵ are each independently selected from H or Halogen.
Item 2. The compound for use of item 1 wherein R³ is trifluoromethyl; and R, R⁴ and R⁵ are H.
Item 3. The compound for use of item 1, wherein the compound is N-[1-[(3,4-difluorophenyl)methyl]-4-piperidinyl]-6-(trifluoromethyl)-3-pyridazinamine, or a compound having the following formula (II) 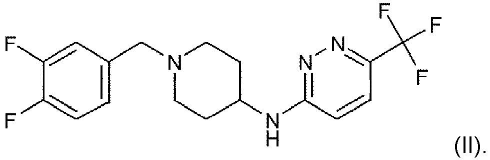
Item 4. The compound for use of item 1, wherein the compound is *N*-[I-(4-fluorobenzyl)piperidin-4-yl]-6-(trifluoromethyl)pyridazin-3-amine, or is a compound according to formula (III) 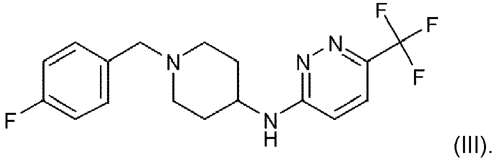
Item 5. The compound for use of item 1, wherein the compound is *N*-[I-(4-fluorobenzyl)piperidin-4-yl]-6-(trifluoromethyl)pyridazin-3-amine, or is a compound according to formula (IV) 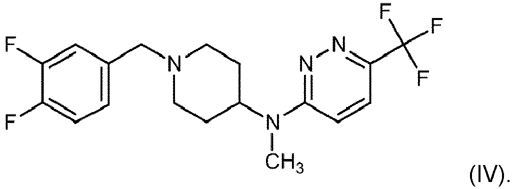
Item 6. The compound for use of item 1, wherein the compound is *N*-[I-(4-fluorobenzyl)piperidin-4-yl]-6-(trifluoromethyl)pyridazin-3-amine, or is a compound according to formula (V) 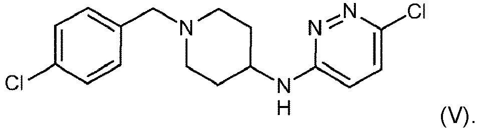
Item 7. The compound for use of item 1, wherein the compound is *N*-[I-(4-fluorobenzyl)piperidin-4-yl]-6-(trifluoromethyl)pyridazin-3-amine, or is a compound according to formula (VI) 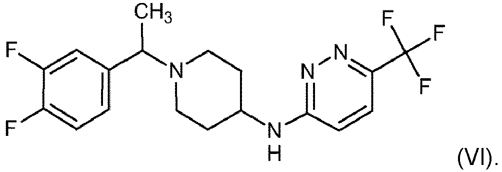
Item 8. The compound for use of any one of the preceding items, wherein the compound is in salt format, and preferably wherein the salt is a dihydrochloride salt.
Item 9. The compound for use of any one of the preceding items, wherein the compound interacts with a Kv7 potassium channel and thereby increases ion flux across the Kv7 potassium channel.
Item 10. The compound for use of any one of items 1 to 9, wherein the compound is an Kv7 agonist, preferably a Kv7.2- and Kv7.3-agonist.
Item 11. The compound for use of any one of the preceding items, wherein the compound retains a capability to interact or at stay in contact with at least one of (preferably all of) residues F104, L242, F304, L312, I300, P308, S303, W236, L299, F305, F240 as a Kv7.2 (SEQ ID NO: 1) binding site when bound to Kv7.2.
Item 12. The compound for use of any one of the preceding items, wherein the Kv7 associated disorder is characterized by the presence of seizures or an epileptic disorder, such as convulsive seizures, in particular, said Kv7 associated disorder is one selected from epilepsy, neonatal spasms, pain, migraine, a disorder of neurotransmitter release, a smooth muscle contractility disorder, a dyskinesia, dystonia, mania, a hearing disorder, neuropathic pain, inflammatory pain, persistent pain, cancer pain, postoperative pain, anxiety, substance abuse, schizophrenia, a bladder disorder, a vasculature disorder, tinnitus, frontotemporal dementia (FTD), familial FTD, or amyotrophic lateral sclerosis.
Item 13. The compound for use of any one of the preceding items, wherein the subject suffers from a seizure inducing disease, and wherein the administration of the compound to the subject reduces the occurrence, prevalence, frequency, duration and/or severity of seizures.
Item 14. **A pharmaceutical composition,** comprising a compound recited in any one of items 1 to 13 in an effective amount to agonise one or more Kv7 potassium channels in a subject, together with a pharmaceutically acceptable carrier and/or excipient.
Item 15. The pharmaceutical composition of item 14, for use as recited in any one of items 1 to 13, preferably for use in the treatment of a disorder characterized by the occurrence of convulsive seizures, such as epilepsy, more preferably wherein the pharmaceutical composition is for use as ASM.

### BRIEF DESCRIPTION OF THE FIGURES AND SEQUENCES

The figures show:

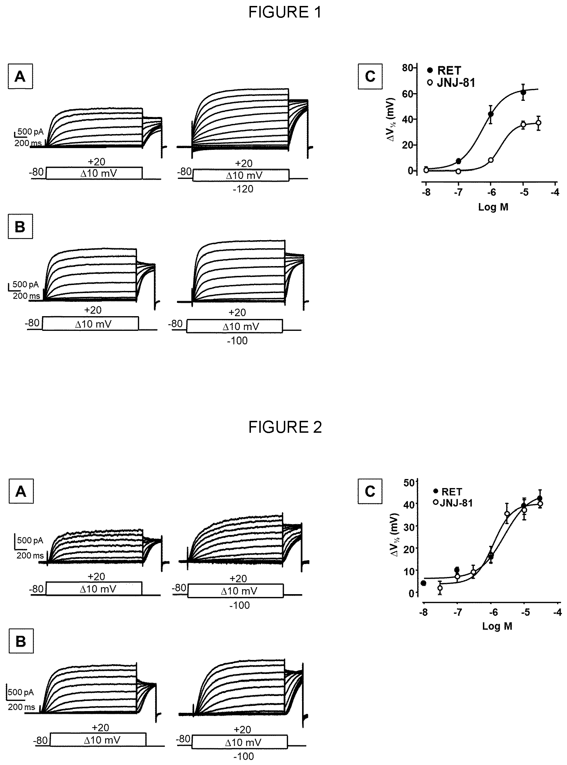

**Figure 1****:** shows **(A)** representative macroscopic current traces recorded from a CHO cell expressing Kv7.3 A315T subunit in response to the indicated voltage protocol before (left) and after (right) application of 1 µM retigabine. Current scale, 500 pA; time scale, 0.2 s. **(B)** Representative macroscopic current traces recorded from a CHO cell expressing Kv7.3 A315T subunit in response to the indicated voltage protocol before (left) and after (right) application of 1 µM JNJ-37822681. Current scale, 500 pA; time scale, 0.2 s. **(C)** Dose-response curves of retigabine (RET, black dots) and JNJ-37822681 (white dots) on Kv7.3 A315T currents. Solid lines represent fits of the experimental data to the four-parameter logistic equation used to estimate EC₅₀ values.
**Figure 2****:** shows **(A)** Representative macroscopic current traces recorded from a CHO cell expressing Kv7.2/7.3 subunit in response to the indicated voltage protocol before (left) and after (right) application of 1 µM retigabine. Current scale, 500 pA; time scale, 0.2 s. **(B)** Representative macroscopic current traces recorded from a CHO cell expressing Kv7.2/7.3 subunit in response to the indicated voltage protocol before (left) and after (right) application of 1 µM JNJ-37822681. Current scale, 500 pA; time scale, 0.2 s. **(C)** Dose-response curves of retigabine (RET, black dots) and JNJ-37822681 (white dots) on Kv7.2/7.3 currents. Solid lines represent fits of the experimental data to the four-parameter logistic equation used to estimate EC₅₀ values.

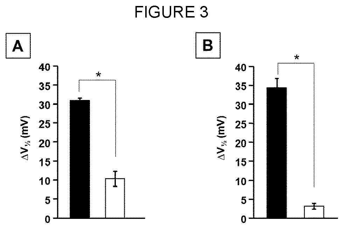

**Figure 3****:** shows **(A)** Effect of the application of 10 µM retigabine on the V_{½} shift (ΔV_{½}) in the homomeric Kv7.2 wild type (black) or mutant W236L (white) channels. **(B)** Effect of the application of 10 µM JNJ-37822681 on the V_{½} shift (ΔV_{½}) in the homomeric Kv7.2 wild type (black) or mutant W236L (white) channels. * indicates values significantly different (p<0.05) from respective controls
**Figure 4****:** shows binding sites identified using the PDB structure with PDB code 7cr2.

The sequences show:
**SEQ ID NOs. 1** shows the amino acid sequence of the human Potassium voltage-gated channel subfamily KQT member 2:

### EXAM PLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: High throughput screening of the repurposing library

To identify repurposed compounds acting as novel and safer Kv7.2/Kv7.3 channel openers, the Fraunhofer repurposing library was screened using a cell-based high throughput screening (HTS).

The Fraunhofer repurposing library is a collection deriving from the Broad Repurposing Hub comprising about 5600 bioactive molecules, whose largest majority is clinically approved for the treatment of neurological/psychiatric, infectious, cardiological, and endocrine diseases.

The Fraunhofer repurposing library was initially screened in a cellular model represented by Chinese hamster ovary cells (CHO) stably transfected with a variant of the Kv7.3 channel (carrying the mutation A315T to enhance current size and increase the signal-to-noise ratio). The Kv7.3 A315T cell line was used in a fluorescence-based thallium flux assay (FluxOR, Thermofisher) optimized as a read-out for Kv7 channel function, using retigabine as a positive control.

Compounds in the Fraunhofer repurposing library were tested in Kv7.3 A315T-expressing cells at 10 µM and their Kv7 opening effect was compared to that observed with 10 µM retigabine. Among 5600 compounds, 59 showed a relative percentage of activation ≥ 20% compared to retigabine and were selected as hits. Retested in the same assay, twelve compounds showed a reproducible activity and were therefore subsequently profiled for their potency in dose-response experiments, using a ten-point dose-response curve from 0.5 nM to 10 µM. Compound JNJ-37822681 emerged as the most potent newly-identified Kᵥ7.3 A315T channel opener; when tested in the FluxOR assay, its potency and maximal efficacy was similar when compared to retigabine (EC₅₀ₛ were 0.30 µM for retigabine and 0.14 µM for JNJ-37822681; maximum activities were 100% for retigabine and 83.8% for JNJ-37822681).

### Example 2: Electrophysiological characterization of JNJ-37822681 as Kv7 activator

JNJ-37822681 efficacy in opening Kv7 channels was further investigated through the whole-cell patch-clamp electrophysiological technique, the gold standard technique for investigating ion channels activity and pharmacological modulation. In CHO cells transiently transfected with Kᵥ7.3 A315T cDNA (Fig.1), perfusion with 1 µM retigabine induced a leftward shift of the channel voltage-dependence of activation (ΔV_{1/2}) of about 10 mV and a slight increase in the maximal current density, as previously reported; qualitatively similar effects were also observed with 1 µM JNJ-37822681 (Fig. 1).

In order to better evaluate the quantitative differences occurring in Kv7-opening ability between retigabine, and JNJ-37822681, dose-response experiments (0.01-30 µM) were performed to calculate EC50s using the functional parameters of ΔV_{1/2} (Figure 1C). In Kv7.3 A315T-transfected cells, JNJ-37822681 was slightly less potent and less effective than retigabine; the EC₅₀ₛ were 2.0±0.03 µM and 0.6±0.1 µM, whereas the maximal ΔV_{1/2} was 37.0±5.4 mV and 60.9±6.1 mV for JNJ-37822681 and retigabine, respectively (p<0.05, n=3-9, Fig.1, panel C).

Most importantly, when both compounds were tested in CHO cells expressing Kv7.2+Kv7.3 channels, representing a more physiological channel configuration, both compounds showed similar efficacy and potency; indeed, the calculated EC₅₀ₛ were 1.2 ± 0.3 µM for JNJ-37822681 and 2.5 ± 1.8 µM for retigabine (p>0.05, n = 3-8; Fig. 2 A, B, C).

### Example 3: Binding site of JNJ-37822681 in Kv7.2

To better characterize the mechanism of action of JNJ-37822681 on Kv7 channels, the molecular identity of its binding site was investigated. Currently available Kv7 activators are known to bind to two distinct sites in Kv7 channels, one located in the voltage-sensing domain and recognized by benzamides, the other located in the pore domain and occupied by retigabine.ln the latter, a H-bonding between the carbamate group of retigabine and the indole nitrogen atom of a tryptophan (W236 in Kv7.2) within the pore domain of Kv7.2-Kv7.5 channels is essential for the Kv7.2-Kv7.5 opening effect. This tryptophan residue acts as a hydrogen bond donor (HBD) with the carbamate group of retigabine acting as a hydrogen bond acceptor (HBA). In fact, the W236L mutation in Kv7.2 largely abolishes retigabine-induced K7.2 activation.

*In silico* simulations of JNJ-37822681 docking performed using the cryoEM structure of Kv7.2 showed that the nitrogen atom in the piperazine ring of JNJ-37822681 could act as acceptor of an H-bond donated by W236, therefore performing a similar interaction as the retigabine carbamate group. In addition, two phenylalanine residues (F240 and F305) in Kv7.2, which are known to interact with the parafluoro phenyl moiety of retigabine, also interacted with the difluorophenyl moiety in JNJ-37822681. Both these observations suggest that similar structural determinants are involved in JNJ-37822681 and retigabine stabilization in Kv7.2 subunits, thus arguing in favour of a largely overlapping binding site.

Functional experiments fully supported the docking simulations data and corroborated the hypothesis that JNJ-37822681 binds Kv7 channels at a site similar to that of retigabine; in fact, JNJ-37822681 (10 µM) failed to activate retigabine-insensitive Kv7.2 channels carrying the W236L mutation (Fig. 3).

### Example 4: Anticonvulsant effects of JNJ-37822681 in two mouse models of seizures_

Electrophysiological experiments showed that JNJ-37822681 is a new Kv7.2/7.3 channels opener with a mechanism of action similar to retigabine. Since activation of Kv7.2/7.3 channels is well-known to exert antiseizure effects *in vivo,* the possible anticonvulsant activity of JNJ-37822681 was investigated.

JNJ-37822681 was tested in two animal models of acute seizures widely used for the evaluation of new ASMs: 1. a mouse model of generalized myoclonic seizures such as acute exposure to the GABA_{A} receptor antagonist pentylenetetrazol (PTZ); 2. audiogenic seizures induced in genetically epilepsy-prone DBA/2 mice. In both models, retigabine effectiveness in reducing seizures severity/frequency is well described; in addition, other newer Kv7.2/7.3 activators were effective in counteracting PTZ-induced seizures or sound-induced seizures in DBA/2 mice, thus confirming the efficacy of these two animal models for evaluating the anticonvulsant efficacy of Kv7 channel activators.

In both animal models, retigabine or JNJ-37822681 were administered i.p, 30 minutes before seizures induction with PTZ or sounds.

In the PTZ model, retigabine (5-30 mg/kg) was effective in reducing the severity of both clonic and tonic phases of seizures, with EC₅₀s of 13 and 9 mg/Kg, respectively. JNJ-37822681 (10-60 mg/kg) was effective in controlling both clonic and tonic phases of seizures with a potency comparable to that of retigabine (EC₅₀s were 15 mg/Kg for clonus and 9 mg/Kg for tonus). When animals were pretreated with the Kv7 antagonist XE-991 (i.p, 3 mg/Kg), both retigabine and JNJ-37822681 anticonvulsant effects were reduced (Table 1) thus proving that JNJ-37822681 anticonvulsant effect was mediated by the activation of XE-991-sensitive Kv7 channels.

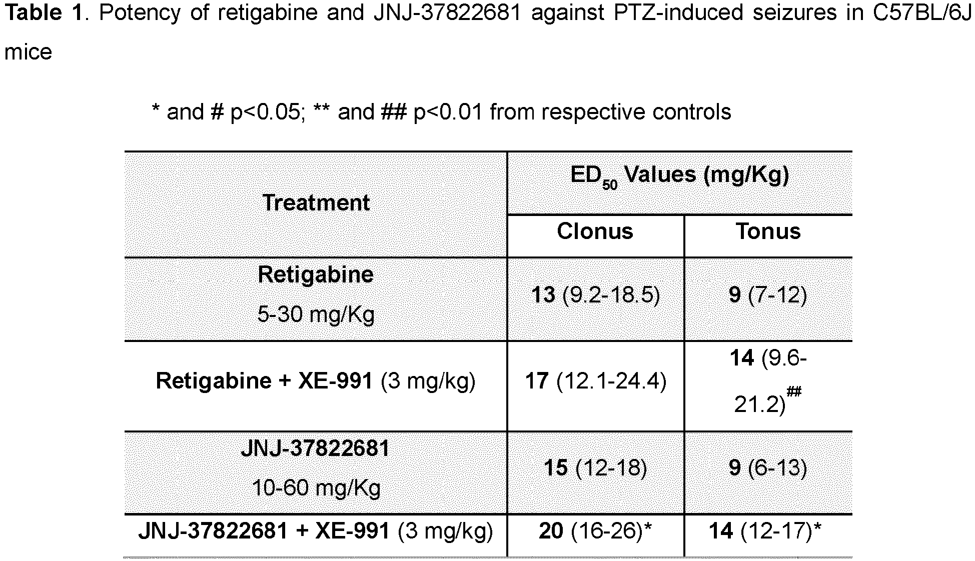

In the DBA/2 model, retigabine (3-20 mg/Kg) dose-dependently reduced sound-induced seizures, showing a protectant effect against wild running, clonic and tonic phases with calculated EC₅₀ of 12, 7 and 4.6 mg/kg, respectively. Compared to retigabine, JNJ-37822681 (5-40 mg/Kg) showed slightly higher potency in reducing wild running, clonic and tonic phases of seizures, with calculated EC₅₀ of 11, 6 and 3 mg/kg, respectively. Pretreatment with the Kv7 antagonist XE-991 (i.p, 3 mg/Kg) reverted the anticonvulsant effect of both retigabine and JNJ-37822681, proving again that the antiseizures effect observed with JNJ-37822681 is due to its Kv7 opening effect. Results are summarized in Table 2.

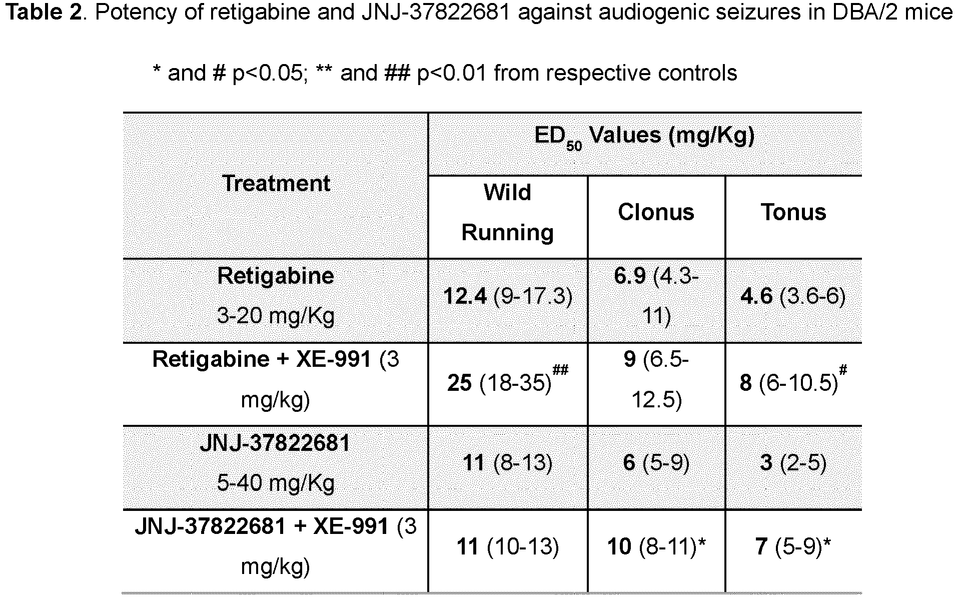

## Claims

1. **A compound for use in the treatment of a Kv7 associated disorder** in a subject, wherein the Kv7 associated disorder is **characterized by** the occurrence of seizures and/or is an epileptic disorder; and wherein the compound is selected from a compound of formula (I), or a pharmaceutically acceptable salt, hydrate or solvate thereof, or a stereoisomeric form thereof, 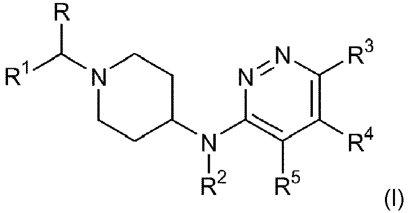 wherein
R is H or C₁₋₆alkyl, in particular methyl;
R¹ is phenyl; phenyl substituted with 1, 2 or 3 substituents each independently selected from the group consisting of H, halogen, cyano, C₁₋₄-alkyl, C₁₋₄-alkyloxy, perfluoro-C₁₋₄-alkyl, diC₁₋₄-alkylamino, in particular 4-fluorophenyl or 3 ,4-difiuorophenyl; thienyl; thienyl substituted with 1 or 2 substituents selected from the group consisting of halo and C₁₋₄-alkyl; C₃₋₈-cycloalkyl; or C₅₋₇-cycloalkenyl;
R² is H or C₁₋₆-alkyl;
R³ is halogen, C₁₋₄-alkyl or perfluoro-C₁₋₄-alkyl; and
R⁴ and R⁵ are each independently selected from H or Halogen.

2. The compound for use of claim 1 wherein R³ is trifluoromethyl; and R, R⁴ and R⁵ are H.

3. The compound for use of claim 1, wherein the compound is N-[1-[(3,4-difluorophenyl)methyl]-4-piperidinyl]-6-(trifluoromethyl)-3-pyridazinamine, or a compound having the following formula (II)

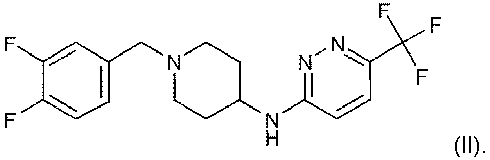

4. The compound for use of claim 1, wherein the compound is *N*-[I-(4-fluorobenzyl)piperidin-4-yl]-6-(trifluoromethyl)pyridazin-3-amine, or is a compound according to formula (III)

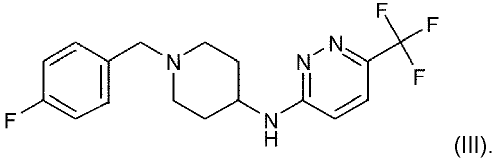

5. The compound for use of claim 1, wherein the compound is *N*-[I-(4-fluorobenzyl)piperidin-4-yl]-6-(trifluoromethyl)pyridazin-3-amine, or is a compound according to formula (IV)

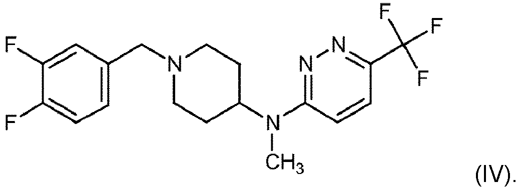

6. The compound for use of claim 1, wherein the compound is *N*-[I-(4-fluorobenzyl)piperidin-4-yl]-6-(trifluoromethyl)pyridazin-3-amine, or is a compound according to formula (V)

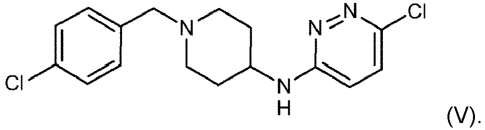

7. The compound for use of claim 1, wherein the compound is *N*-[I-(4-fluorobenzyl)piperidin-4-yl]-6-(trifluoromethyl)pyridazin-3-amine, or is a compound according to formula (VI)

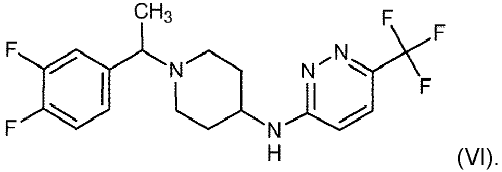

8. The compound for use of any one of the preceding claims, wherein the Kv7 associated disorder is a Kv7.2- and/or Kv7.3-associated disorder.

9. The compound for use of any one of the preceding claims, wherein the compound interacts with a Kv7 potassium channel and thereby increases ion flux across the Kv7 potassium channel.

10. The compound for use of any one of claims 1 to 9, wherein the compound is an Kv7 agonist, preferably a Kv7.2- and Kv7.3-agonist.

11. The compound for use of any one of the preceding claims, wherein the compound retains a capability to interact or at stay in contact with at least one of (preferably all of) residues F104, L242, F304, L312, I300, P308, S303, W236, L299, F305, F240 as a Kv7.2 (SEQ ID NO: 1) binding site when bound to Kv7.2.

12. The compound for use of any one of the preceding claims, wherein the Kv7 associated disorder is one selected from epilepsy, neonatal spasms, pain, migraine, a disorder of neurotransmitter release, a smooth muscle contractility disorder, a dyskinesia, dystonia, mania, a hearing disorder, neuropathic pain, inflammatory pain, persistent pain, cancer pain, postoperative pain, anxiety, substance abuse, schizophrenia, a bladder disorder, a vasculature disorder, tinnitus, frontotemporal dementia (FTD), familial FTD, or amyotrophic lateral sclerosis.

13. The compound for use of any one of the preceding claims, wherein the subject suffers from a seizure inducing disease, and wherein the administration of the compound to the subject reduces the occurrence, prevalence, frequency, duration and/or severity of seizures.

14. **A pharmaceutical composition,** comprising a compound recited in any one of claims 1 to 13 in an effective amount to agonise one or more Kv7 potassium channels in a subject, together with a pharmaceutically acceptable carrier and/or excipient.

15. The pharmaceutical composition of claim 14, for use as recited in any one of claims 1 to 13, preferably for use in the treatment of a disorder **characterized by** the occurrence of convulsive seizures, such as epilepsy, more preferably wherein the pharmaceutical composition is for use as ASM.
